# EUROPÄISCHE PATENTSCHRIFT

(11) **EP 3 349 217 B1**
(45) Veröffentlichungstag und Bekanntmachung des Hinweises auf die Patenterteilung: **30.06.2021**
(21) Anmeldenummer: 17192188.5
(22) Anmeldetag: 20.09.2017
(51) Int. Cl.: G16H 20/10

(54) **VERFAHREN UND DATENVERARBEITUNGSEINHEIT ZUM ERMITTELN EINES PATIENTENSPEZIFISCHEN PHARMAKOKINETISCHEN PARAMETERS**
METHOD AND DATA PROCESSING UNIT FOR DETERMINING A PATIENT-SPECIFIC PHARMACOKINETIC PARAMETER
PROCÉDÉ ET UNITÉ DE TRAITEMENT DE DONNÉES PERMETTANT DE DÉTERMINER UN PARAMÈTRE PHARMACOCINÉTIQUE PROPRE AU PATIENT

(43) Veröffentlichungstag der Anmeldung: 18.07.2018
(73) Patentinhaber: Siemens Healthcare GmbH, 91052 Erlangen (DE)
(72) Erfinder: Schmidt, Sebastian, 91085 Weisendorf (DE)

(56) Entgegenhaltungen:
- WO-A1-2017/049197
- US-A1- 2007 196 479
- US-A1- 2009 210 209
- US-A1- 2016 335 412
- PATRICK POULIN ET AL: "PHRMA CPCDC initiative on predictive models of human pharmacokinetics, part 5: Prediction of plasma concentration-time profiles in human by using the physiologically-based pharmacokinetic modeling approach", JOURNAL OF PHARMACEUTICAL SCIENCES, Bd. 100, Nr. 10, 1. Oktober 2011 (2011-10-01), Seiten 4127-4157, XP055460582, US ISSN: 0022-3549, DOI: 10.1002/jps.22550
- Anonymous: "ADME", Wikipedia, 16. August 2017 (2017-08-16), XP055461201, Gefunden im Internet: URL:https://en.wikipedia.org/w/index.php?t itle=ADME&oldid=795782470 [gefunden am 2018-03-20]
- Anonymous: "Machine learning", Wikipedia, 18 September 2017 (2017-09-18), XP055600243, Retrieved from the Internet: URL:https://en.wikipedia.org/w/index.php?t itle=Machine_learning&oldid=801298587 [retrieved on 2019-06-27]
- WORKIE D W ET AL: "Quantification of dynamic contrast-enhanced MR imaging of the knee in children with juvenile rheumatoid arthritis based on pharmacokinetic modeling", MAGNETIC RESONANCE IMAGING, ELSEVIER SCIENCE, TARRYTOWN, NY, US, vol. 22, no. 9, 1 November 2004 (2004-11-01), pages 1201-1210, XP004679806, ISSN: 0730-725X, DOI: 10.1016/J.MRI.2004.09.006
- Krishna Juluru ET AL: "Diagnostic accuracy of intracellular uptake rates calculated using dynamic Gd-EOB-DTPA-enhanced MRI for hepatic fibrosis stage : DCE-MRI for Liver Fibrosis Stage", JOURNAL OF MAGNETIC RESONANCE IMAGING, vol. 45, no. 4, 16 August 2016 (2016-08-16), pages 1177-1185, XP055694876, US ISSN: 1053-1807, DOI: 10.1002/jmri.25431

## Beschreibung

Die Erfindung betrifft ein Verfahren zum Ermitteln eines patientenspezifischen pharmakokinetischen Parameters, ein Verfahren zum Ermitteln eines patientenspezifischen Medikationsparameters und ein Verfahren zum Trainieren eines Bilddatenverarbeitungsalgorithmus. Die Erfindung betrifft ferner eine Datenverarbeitungseinheit, ein Computerprogrammprodukt und ein computerlesbares Medium.

Medikamente wirken auf verschiedene Personen sehr unterschiedlich. Ein wesentlicher Grund dafür ist die spezifische Pharmakokinetik, also die Aufnahme, Verteilung, Metabolisierung und Ausscheidung des Medikaments im Körper (auch ADME genannt, für Absorption, Distribution, Metabolization and Excretion). Daher werden Medikamente umfangreich auf ihre Pharmakokinetik getestet, allerdings nur an größeren Populationen. Damit ist im Mittel das Verhalten eines Medikaments im Körper bekannt, aber nicht im konkreten Patienten. In vielen Situationen wäre dies aber notwendig. Zum Beispiel verteilen sich lipophile Wirkstoffe zwischen wässrigem und fetthaltigem Gewebe ungleichmäßig. Ist der Fettgehalt im Körper groß, ist der Spiegel im Blut trotz ausreichender Dosis gering und die Wirkung fehlt. Wird hier nur nach Körpergewicht dosiert, kommt es andersrum bei Patienten mit großer Muskelmasse zur Überdosierung - ein Patient mit großem Körpergewicht kann eine große Muskelmasse haben, aber auch viel Fettgewebe. Ähnlich ist es bei Problemen in der Metabolisierung und Ausscheidung, zum Beispiel aufgrund von Leber- oder Nierenschäden oder nach Operationen dieser Organe. Hier kann schnell eine Überdosierung entstehen.

In der Tat hängt der erreichte Blut- und Gewebespiegel eines Medikaments bei gegebener Dosierung in komplexer Art und Weise von der Funktion mehrerer Organe ab, die sich vor allem im Brust- und Bauchraum befinden. Dies ist immer dann problematisch, wenn ein Medikament eine geringe therapeutische Breite hat, also der Abstand von therapeutischer Dosis zu toxischer Dosis sehr klein ist. Dies ist zum Beispiel bei onkologischen Therapien der Fall, bei Antiepileptika und einigen Psychopharmaka.

Es existieren Computer-Modelle zu ADME-Simulationen. Diese erlauben es, für einen noch wenig charakterisierten Wirkstoff, das voraussichtliche Verhalten zu charakterisieren. Diese verwenden jedoch immer "Modellmenschen" und sind nicht geeignet, eine konkrete Vorhersage für einen einzelnen Menschen zu treffen. Des Weiteren wird die richtige Dosis oft durch Ausprobieren bestimmt. Dazu wird zunächst eine kleine Dosis gegeben, der Blutspiegel bestimmt und dann Schritt für Schritt bis zum korrekten Blutspiegel erhöht. Dieses Verfahren dauert aber lange, ist aufgrund der Laboruntersuchungen kostspielig und für Notfälle nicht geeignet. Alternativ kann die Dosis anhand bekannter Parameter (Gewicht, Körperoberfläche) sehr grob abgeschätzt werden.

WO 2017/049197 A1 offenbart ein Verfahren zur Abschätzung einer Arzneimittelabgabe an einem Zielgewebe.

US 2009/210209 A1 offenbart ein Verfahren zur Simulation einer Wirkung von pharmazeutischen Substanzen in einem lebenden Körper.

WORKIE D W ET AL: "Quantification of dynamic contrastenhanced MR imaging of the knee in children with juvenile rheumatoid arthritis based on pharmacokinetic modeling", MAGNETIC RESONANCE IMAGING, ELSEVIER SCIENCE, TARRYTOWN, NY, US, Bd. 22, Nr. 9, 1. November 2004, offenbart eine Quantifizierung einer dynamischen kontrastmittelverstärkten MR-Bildgebung basierend auf pharmakokinetischen Modellen.

Krishna Juluru ET AL: "Diagnostic accuracy of intracellular uptake rates calculated using dynamic Gd-EOB-DTPA-enhanced

MRI for hepatic fibrosis stage: DCE-MRI for Liver Fibrosis Stage", JOURNAL OF MAGNETIC RESONANCE IMAGING, Bd. 45, Nr. 4, 16. August 2016, offenbart eine Bewertung einer diagnostischen Genauigkeit von intrazellulären Aufnahmeraten mittels MRT für das Stadium der Leberfibrose.

Die Erfindung hat die Aufgabe, eine verbesserte patientenspezifische pharmakokinetische Parametrierung in Bezug auf eine hepatische Elimination zu ermöglichen. Jeder der Gegenstände der unabhängigen Ansprüche löst jeweils diese Aufgabe. In den abhängigen Ansprüchen sind weitere vorteilhafte Aspekte der Erfindung berücksichtigt.

Die Erfindung betrifft ein Verfahren zum Ermitteln eines patientenspezifischen pharmakokinetischen Parameters für ein Medikament, wobei das Verfahren die folgenden Schritte umfasst:
- Bereitstellen von allgemeinen pharmakokinetischen Daten des Medikaments, insbesondere mittels einer pharmakokinetischen Datenbank,
- Bereitstellen von radiologischen medizinischen Bilddaten eines Patienten,
- Ermitteln des patientenspezifischen pharmakokinetischen Parameters für das Medikament basierend auf den medizinischen Bilddaten und den allgemeinen pharmakokinetischen Daten des Medikaments,
- wobei ein Bildverarbeitungsergebnis basierend auf den medizinischen Bilddaten des Patienten mittels eines Bilddatenverarbeitungsverfahrens ermittelt wird, wobei das Bildverarbeitungsergebnis eine Textur einer Leber betrifft,
- wobei der patientenspezifische pharmakokinetische Parameter basierend auf dem Bildverarbeitungsergebnis ermittelt wird, wobei der patientenspezifische pharmakokinetische Parameter für das Medikament eine Eliminationsgeschwindigkeit einer hepatischen Elimination des Medikaments in einer Reaktion nullter Ordnung ist,
- wobei der patientenspezifische pharmakokinetische Parameter für das Medikament unter Verwendung eines trainierten Bilddatenverarbeitungsalgorithmus ermittelt wird, wobei aus der Textur der Leber über maschinelles Lernen eine Elimination pro Volumeneinheit berechnet wird, wobei ein Verfahren des maschinellen Lernens verwendet wird, welches anhand von Bilddatensätzen mit bekannter Pharmakokinetik trainiert wurde,
- wobei mittels einer Segmentierung ein Gesamtvolumen der Leber bestimmt wird und
- wobei aus der Elimination pro Volumeneinheit und dem Gesamtvolumen der Leber der patientenspezifische pharmakokinetische Parameter für das Medikament ermittelt wird.

Das Ermitteln des patientenspezifischen pharmakokinetischen Parameters für das Medikament basierend auf den medizinischen Bilddaten und den allgemeinen pharmakokinetischen Daten des Medikaments kann insbesondere automatisch, beispielsweise mittels eines Prozessorsystems, erfolgen. Unter dem Ermitteln des patientenspezifischen pharmakokinetischen Parameters kann insbesondere ein Ermitteln eines Werts des patientenspezifischen pharmakokinetischen Parameters verstanden werden.

Viele Patienten, die Medikamente bekommen sollen, erhalten ohnehin radiologische Aufnahmen, speziell Schnittbildaufnahmen mittels Computertomographie (CT) oder Magnetresonanztomographie (MRT). Daher liegen in vielen Fällen für solche Patienten medizinische Bilddaten vor, speziell des Bauchraums (Abdomen) und des Brustraums (Thorax). Heutzutage sind durch elektronische Patientenakten diese medizinischen Bilddaten auch gut zugänglich, so dass für die Ermittlung des patientenspezifischen pharmakokinetischen Parameters nicht eine zusätzliche medizinische Bildgebungsuntersuchung des Patienten erforderlich ist. Aus den medizinischen Bilddaten können Informationen zur Pharmakokinetik eines Medikaments in diesem Patienten gewonnen werden.

Die Morphologie kann sich insbesondere auf eine Größe, eine Form, eine Dimension, eine Umrandung, eine Grenzschicht oder ähnliches eines Körperbereichs, insbesondere eines Organs, beziehen. Die Textur kann sich insbesondere auf eine Feinstruktur und/oder eine Musterstruktur der Grauwertverteilung in einer Darstellung des Körperbereichs, insbesondere des Organs, in den medizinischen Bilddaten beziehen.

Eine Ausführungsform der Erfindung sieht vor, dass das Bilddatenverarbeitungsverfahren einen Bildverarbeitungsschritt umfasst, welcher aus der Gruppe gewählt ist, die aus einer Segmentierung, einer Texturanalyse, einer Perfusionsbestimmung, einer Stoffwechselaktivitätsbestimmung und Kombinationen davon besteht.

Eine Ausführungsform der Erfindung sieht vor, dass basierend auf den pharmakokinetischen Daten ein Körperbereich ausgewählt wird, welcher an einem pharmakokinetischen Prozess des Medikaments beteiligt ist, und/oder dass das Bilddatenverarbeitungsverfahren basierend auf dem ausgewählten Körperbereich ausgewählt und/oder angepasst wird.

Bei einem Körperbereich kann es sich insbesondere um ein oder mehrere Organe und/oder Kompartimente handeln. Beispielsweise sind bei strikt hepatischer Elimination die Leber und die Gallenwege relevant. Beispielsweise sind bei renaler Elimination die Niere und die Harnwege relevant.

Eine Ausführungsform der Erfindung sieht vor, dass die allgemeinen pharmakokinetischen Daten eine oder mehrere pharmakokinetische Eigenschaften des Medikaments betreffen, welche aus der Gruppe gewählt sind, die aus einer Applikation (Verabreichung), einer Bioverfügbarkeit, einer Lipophilität, einem Verteilungsraum, einem Eliminationsweg und Kombinationen davon besteht. Unter allgemeinen pharmakokinetischen Daten des Medikaments können insbesondere pharmakokinetische Daten des Medikaments verstanden werden, die nicht patientenspezifisch sind, insbesondere für alle Patienten gleich sind.

Der pharmakokinetische Parameter ist sowohl medikamentespezifisch als auch patientenspezifisch, insbesondere spezifisch für ein Organ des Patienten, welches an dem pharmakokinetischen Prozess des Medikaments beteiligt ist. Insbesondere kann der pharmakokinetische Parameter von einer Morphologie des Organs des Patienten abhängen. Für bestimmte Körperbereiche und entsprechende pharmakokinetische Prozesse kann es zutreffen, dass der pharmakokinetische Parameter nur relativ schwach von dem Medikament abhängt, also nur geringfügig medikamentespezifisch ist. Zum Beispiel verhalten sich praktisch alle Medikamente, die in der Leber oxidativ abgebaut werden, ähnlich.

Für einen oxidativen pharmakokinetischen Prozess in der Leber sind insbesondere das Volumen, die Perfusion und die Textur relevant. Dabei ist der pharmakokinetische Parameter proportional zum Volumen der Leber und umso kleiner, je schlechter die Perfusion ist. Zwischen der Textur der Leber und dem pharmakokinetischen Parameter besteht ein nicht-linearer Zusammenhang.

Da die Ermittlung des patientenspezifischen pharmakokinetischen Parameters in der Regel komplex und nicht-linear von mehreren Größen abhängt, bieten sich hierzu Verfahren aus dem Bereich des maschinellen Lernen an, welche anhand von Bilddatensätzen mit bekannter Pharmakokinetik trainiert werden. Der trainierte Bilddatenverarbeitungsalgorithmus kann insbesondere zum Ermitteln des patientenspezifischen pharmakokinetischen Parameters für das Medikament basierend auf den medizinischen Bilddaten und/oder dem Bildverarbeitungsergebnis ausgebildet sein. Beispielsweise kann der trainierte Bilddatenverarbeitungsalgorithmus basierend auf den medizinischen Bilddaten das Bildverarbeitungsergebnis ermittelt und/oder basierend auf dem Bildverarbeitungsergebnis den patientenspezifischen pharmakokinetischen Parameter ermitteln.

Ergänzend zu dem trainierten Bilddatenverarbeitungsalgorithmus können insbesondere manuelle und/oder regelbasierte Bildverarbeitungsschritte ausgeführt werden. Beispielsweise kann aus der Textur über maschinelles Lernen eine Elimination pro Volumeneinheit berechnet werden. Mittels einer Segmentation, die nicht notwendigerweise auf maschinellem Lernen basieren muss, kann das Gesamtvolumen bestimmt werden. Aus der Elimination pro Volumeneinheit und dem Gesamtvolumen kann dann ein patientenspezifischer pharmakokinetischer Parameter für die Elimination ermittelt werden.

Die Erfindung betrifft ferner ein Verfahren zum Ermitteln eines patientenspezifischen Medikationsparameters für ein Medikament, wobei das Verfahren die folgenden Schritte umfasst:
- Ermitteln zumindest eines patientenspezifischen pharmakokinetischen Parameters für das Medikament mittels eines Verfahrens zum Ermitteln eines patientenspezifischen pharmakokinetischen Parameters nach einem oder mehreren der in dieser Anmeldung offenbarten Aspekte,
- Ermitteln des patientenspezifischen Medikationsparameters basierend auf dem zumindest einen patientenspezifischen pharmakokinetischen Parameter und einem pharmakokinetischen Modell, insbesondere einem ADME-Modell.

Insbesondere kann das Verfahren zum Ermitteln eines patientenspezifischen pharmakokinetischen Parameters mehrmals ausgeführt werden, um alle patientenspezifischen pharmakokinetischen Parameter, die für das pharmakokinetische Modell wesentlich sind, zu ermitteln. Insbesondere können mit dem Verfahren zum Ermitteln eines patientenspezifischen Medikationsparameters mehrere patientenspezifische Medikationsparameter basierend auf dem zumindest einen patientenspezifischen pharmakokinetischen Parameter und dem pharmakokinetischen Modell ermittelt werden. Wenn ein bestimmter patientenspezifischer pharmakokinetischer Parameter nicht aus den vorliegenden medizinischen Bilddaten berechnet werden kann, zum Beispiel weil das Organ nicht abgebildet ist, können stattdessen Standardwerte oder Schätzwerte, zum Beispiel anhand des Körpergewichts, verwendet werden.

Unter einem Medikationsparameter kann insbesondere eine Menge des Medikaments bei einer Medikamente-Applikation und/oder ein Zeitabstand zwischen zwei zeitlich benachbarten Medikamente-Applikationen des Medikaments verstanden werden. Damit kann zum Beispiel ein Medikationsplan erzeugt werden. Insbesondere kann damit angegeben werden, wann der Patient welche Dosis des Medikaments einnehmen muss, damit beispielsweise der Blutspiegel des Medikaments über einen bestimmten Zeitraum relativ konstant in einem definierten Band bleibt und/oder eine gewünschte Konzentration des Medikaments in einem Zielorgan erreicht wird. Auf diese Weise kann das Medikament viel genauer dosiert werden.

Eine Ausführungsform der Erfindung sieht vor, dass der patientenspezifische Medikationsparameter ermittelt wird, indem ein patientenspezifischer zeitlicher Verlauf einer Konzentration des Medikaments, insbesondere im Blut des Patienten, simuliert wird, insbesondere basierend auf einem ADME-Modell simuliert wird.

Eine beispielhafte Anwendung des Verfahrens sieht vor, dass der Arzt dem Patienten ein bestimmtes Medikament verordnet und einen zu erreichenden Spiegel des Medikaments in Blut oder Gewebe festlegt. Diese Verordnung wird in eine elektronische Patientenakte eingetragen. Anhand des verordneten Medikaments und der medizinischen Bilder wird ein pharmakokinetisches Modell patientenspezifisch parametrisiert, so dass die Pharmakokinetik des Medikaments im Körper des Patienten, insbesondere die ADME-Prozesse, simuliert werden können. Daraus wird ein Dosierungsvorschlag automatisch generiert. Diesen kann der Arzt akzeptieren oder verändern. Anschließend wird der Dosierungsvorschlag in die elektronische Patientenakte eingetragen und zum Beispiel an den Patienten und/oder an die Apotheke übermittelt. Wird im Laufe der Medikation eine laborchemische Kontrolle des Blutspiegels vorgenommen, kann diese verwendet werden, um den Bilddatenverarbeitungsalgorithmus und/oder das pharmakokinetische Modell zu optimieren.

Eine Ausführungsform der Erfindung sieht vor, dass das Verfahren zum Ermitteln des patientenspezifischen pharmakokinetischen Parameters für das Medikament ferner die folgenden Schritte umfasst:
- Bereitstellen eines Satzes von Trainingsdatensätzen, wobei jeder Trainingsdatensatz jeweils einen medizinischen Bilddatensatz und einen dem medizinischen Bilddatensatz zugeordneten pharmakokinetischen Parameter aufweist,
- Trainieren des Bilddatenverarbeitungsalgorithmus basierend auf dem Satz von Trainingsdatensätzen und einem Maschinenlernalgorithmus.

Dabei können medizinische Bilddatensätze von unterschiedlichen Patienten und/oder von unterschiedlichen Bildgebungsmodalitäten verwendet werden. Unter einem Maschinenlernalgorithmus wird im Kontext dieser Anmeldung insbesondere ein Algorithmus, der zum Maschinellen Lernen ausgebildet ist, verstanden. Ein Maschinenlernalgorithmus kann beispielsweise mit Hilfe von Entscheidungsbäumen, mathematischen Funktionen und/oder allgemeinen Programmiersprachen realisiert werden. Der Maschinenlernalgorithmus kann beispielsweise zum überwachten Lernen und/oder zum unüberwachten Lernen ausgebildet sein. Der Maschinenlernalgorithmus kann beispielsweise zum tiefen Lernen ("deep learning") und/oder zum bestärkendem Lernen ("reinforcement learning") und/oder zum Marginal Space Learning ausgebildet sein. Insbesondere beim überwachten Lernen kann eine Funktionenklasse verwendet werden, welche beispielsweise auf Entscheidungsbäumen ("decision trees"), einem Random Forest, einer logistischen Regression, einer Support Vector Machine, einem künstlichen neuronalen Netz, einer Kernel-Methode, Bayes-Klassifikatoren oder ähnlichem oder Kombinationen davon basiert. Mögliche Implementierungen des Maschinenlernalgorithmus können beispielsweise künstliche Intelligenz verwenden. Berechnungen, insbesondere beim Trainieren des Bilddatenverarbeitungsalgorithmus, können beispielsweise mittels eines Prozessorsystems ausgeführt werden. Das Prozessorsystem kann beispielsweise einen oder mehrere Prozessoren, insbesondere Grafikprozessoren, aufweisen.

Die Erfindung betrifft ferner eine Datenverarbeitungseinheit, ausgebildet zum Ausführen eines Verfahrens nach einem oder mehreren der in dieser Anmeldung offenbarten Aspekte.

Insbesondere ist hiermit eine Datenverarbeitungseinheit zum Ermitteln eines patientenspezifischen pharmakokinetischen Parameters offenbart, aufweisend:
- eine Pharmakokinetik-Daten-Bereitstellungseinheit, ausgebildet zum Bereitstellen von allgemeinen pharmakokinetischen Daten des Medikaments,
- eine Bilddaten-Bereitstellungseinheit, ausgebildet zum Bereitstellen von medizinischen Bilddaten eines Patienten,
- eine Parameter-Ermittlungseinheit, ausgebildet zum Ermitteln des patientenspezifischen pharmakokinetischen Parameters für das Medikament basierend auf den medizinischen Bilddaten und den allgemeinen pharmakokinetischen Daten des Medikaments.

Insbesondere ist hiermit eine Datenverarbeitungseinheit zum Ermitteln eines patientenspezifischen Medikationsparameters offenbart, aufweisend:
- eine Trainingsdatensatz-Bereitstellungseinheit, ausgebildet zum Bereitstellen eines Satzes von Trainingsdatensätzen, wobei jeder Trainingsdatensatz jeweils einen medizinischen Bilddatensatz und einen dem medizinischen Bilddatensatz zugeordneten pharmakokinetischen Parameter aufweist,
- eine Bilddatenverarbeitungsalgorithmus-Trainingseinheit, ausgebildet zum Trainieren des Bilddatenverarbeitungsalgorithmus basierend auf dem Satz von Trainingsdatensätzen und einem Maschinenlernalgorithmus,
- eine Pharmakokinetik-Daten-Bereitstellungseinheit, ausgebildet zum Bereitstellen von allgemeinen pharmakokinetischen Daten des Medikaments,
- eine Bilddaten-Bereitstellungseinheit, ausgebildet zum Bereitstellen von medizinischen Bilddaten eines Patienten,
- eine Parameter-Ermittlungseinheit, ausgebildet zum Ermitteln des patientenspezifischen pharmakokinetischen Parameters für das Medikament basierend auf den medizinischen Bilddaten und den allgemeinen pharmakokinetischen Daten des Medikaments unter Verwendung des trainierten Bilddatenverarbeitungsalgorithmus,
- eine Medikationsparameter-Ermittlungseinheit, ausgebildet zum Ermitteln des patientenspezifischen Medikationsparameters basierend auf dem zumindest einen patientenspezifischen pharmakokinetischen Parameter und einem pharmakokinetischen Modell.

Die Erfindung betrifft ferner ein Computerprogrammprodukt mit einem Computerprogramm, welches direkt in eine Speichereinrichtung eines Computers ladbar ist, mit Programmabschnitten, um alle Schritte eines Verfahrens nach einem oder mehreren der in dieser Anmeldung offenbarten Aspekte auszuführen, wenn das Computerprogramm in dem Computer ausgeführt wird.

Die Erfindung betrifft ferner ein computerlesbares Medium, auf welchem von einem Computer einlesbare und ausführbare Programmabschnitte gespeichert sind, um alle Schritte eines Verfahrens nach einem oder mehreren der in dieser Anmeldung offenbarten Aspekte auszuführen, wenn die Programmabschnitte von dem Computer ausgeführt werden.

Die Datenverarbeitungseinheit und/oder eine oder mehrere Komponenten der Datenverarbeitungseinheit können von einem Datenverarbeitungssystem gebildet sein. Das Datenverarbeitungssystem kann beispielsweise eine oder mehrere Komponenten in Form von Hardware und/oder eine oder mehrere Komponenten in Form von Software aufweisen.

Das Datenverarbeitungssystem kann beispielsweise zumindest teilweise von einem Cloud-Computing-System gebildet sein.
Das Datenverarbeitungssystem kann beispielsweise ein Cloud-Computing-System, ein Computernetzwerk, ein Computer, ein Tabletcomputer, ein Smartphone oder ähnliches oder eine Kombination davon sein und/oder aufweisen. Die Hardware kann beispielsweise mit einer Software zusammenwirken und/oder mittels einer Software konfigurierbar sein. Die Software kann beispielsweise mittels der Hardware ausgeführt werden. Bei der Hardware kann es sich beispielsweise um ein Speichersystem, ein FPGA-System (Field-programmable gate array), ein ASIC-System (Application-specific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen.

Insbesondere kann eine Komponente einer Datenverarbeitungseinheit nach einem oder mehreren der Aspekte, die in dieser Anmeldung offenbart sind, welche dazu ausgebildet ist, einen gegebenen Schritt eines Verfahrens nach einem oder mehreren der Aspekte, die in dieser Anmeldung offenbart sind, auszuführen, in Form einer Hardware implementiert sein, welche zum Ausführen des gegebenen Schritts konfiguriert ist und/oder welche zum Ausführen einer computerlesbaren Anweisung derart konfiguriert ist, dass die Hardware mittels der computerlesbaren Anweisung zum Ausführen des gegebenen Schritts konfigurierbar ist. Insbesondere kann das Datenverarbeitungssystem einen Speicherbereich, beispielsweise in Form eines computerlesbaren Mediums, aufweisen, in welchem computerlesbare Anweisungen, beispielsweise in Form eines Computerprogramms, gespeichert sind.

Ein Datentransfer zwischen Komponenten des Datenverarbeitungssystems kann beispielsweise jeweils mittels einer geeigneten Datentransfer-Schnittstelle erfolgen. Die Datentransfer-Schnittstelle zum Datentransfer an und/oder von einer Komponente des Datenverarbeitungssystems kann zumindest teilweise in Form von Software und/oder zumindest teilweise in Form von Hardware realisiert sein. Die Datentransfer-Schnittstelle kann beispielsweise zum Abspeichern von Daten in und/oder zum Laden von Daten aus einem Bereich des Speichersystems ausgebildet sein, wobei auf diesen Bereich des Speichersystems eine oder mehrere Komponenten des Datenverarbeitungssystems zugreifen können.

Insbesondere können Daten, beispielsweise medizinische Bilddaten, allgemeine pharmakokinetische Daten oder Trainingsdatensätze, bereitgestellt werden, indem die Daten geladen, beispielsweise aus einem Bereich eines Speichersystems geladen, und/oder erzeugt, beispielsweise mittels einer medizinischen Bildgebungsvorrichtung erzeugt, werden.

Das Computerprogramm ist in das Speichersystem des Datenverarbeitungssystems ladbar und von dem Prozessorsystem des Datenverarbeitungssystems ausführbar. Das Datenverarbeitungssystem kann beispielsweise mittels des Computerprogramms derart ausgebildet sein, dass das Datenverarbeitungssystem die Schritte eines Verfahrens nach einer der Ausführungsformen, die in dieser Anmeldung offenbart sind, ausführen kann, wenn das Computerprogramm von dem Datenverarbeitungssystem ausgeführt wird.

Das Computerprogrammprodukt kann beispielsweise das Computerprogramm sein oder neben dem Computerprogramm mindestens einen zusätzlichen Bestandteil umfassen. Der mindestens eine zusätzliche Bestandteil des Computerprogrammprodukts kann als Hardware und/oder als Software ausgebildet sein. Das Computerprogrammprodukt kann beispielsweise ein Speichermedium, auf dem zumindest ein Teil des Computerprogrammprodukts gespeichert ist, und/oder ein Schlüssel zur Authentifizierung eines Benutzers des Computerprogrammprodukts, insbesondere in Form eines Dongles, aufweisen.

Das Computerprogrammprodukt und/oder das Computerprogramm kann beispielsweise ein Cloud-Anwendungs-Programm aufweisen, welches zum Verteilen von Programmabschnitten des Computerprogramms auf verschiedene Verarbeitungseinheiten, insbesondere verschiedene Computer, eines Cloud-Computing-Systems ausgebildet ist, wobei jede der Verarbeitungseinheiten zum Ausführen eines oder mehrerer Programmabschnitte des Computerprogramms ausgebildet ist.

Auf dem computerlesbaren Medium kann beispielsweise das Computerprogrammprodukt nach einer der Ausführungsformen, die in dieser Anmeldung offenbart sind, und/oder das Computerprogramm nach einer der Ausführungsformen, die in dieser Anmeldung offenbart sind, gespeichert sein. Das computerlesbare Medium kann beispielsweise ein Memorystick, eine Festplatte oder ein sonstiger Datenträger sein, der insbesondere lösbar mit dem Datenverarbeitungssystem verbunden oder fest in das Datenverarbeitungssystem integriert sein kann. Das computerlesbare Medium kann beispielsweise einen Bereich des Speichersystems des Datenverarbeitungssystems bilden.

Im Folgenden wird das Ermitteln von patientenspezifischen pharmakokinetischen Parametern basierend auf medizinischen Bilddaten an verschiedenen Beispielen näher erläutert.

Die Elimination erfolgt typischerweise in einer Reaktion erster Ordnung, dC/dt = -k*C (C= Konzentration des Medikaments, t=Zeit, k=pharmakokinetischer Parameter), oder nullter Ordnung, dC/dt = K, mit konstanter Eliminationsgeschwindigkeit. Ein prominentes Beispiel für eine Eliminationskinetik nullter Ordnung ist Ethanol.

Ethanol hat eine rasche Bioverfügbarkeit von nahezu 100%, daher kann die Absorption hier zur Vereinfachung ignoriert werden. Das Verteilungsvolumen ist in etwa das Körpervolumen, da sich Ethanol in Wasser und Fett gut löst. Gegeben ist die zugeführte Alkoholmenge. Daher wird zuerst die Distribution (D) simuliert. Dazu kann aus Computertomographie-Bilddaten die Körpermasse ermittelt werden, sofern sie nicht vorbekannt ist. Dies funktioniert über den Bauchumfang, Dicke des Fettgewebes und die Abmessungen der Organe relativ gut. Die Blutkonzentration ist dann die aufgenommene Menge / Körpermasse.

Die Metabolisierung und Elimination erfolgt über Leber und einen kleineren Teil über die Lunge mit der Atemluft. Für die Metabolisierung, welche vor allem über die Leber erfolgt, wird das Lebervolumen, Fettgehalt der Leber (über die Hounsfield-Dichte) und die Textur der Leber als Maß für den bindegewebigen Umbau herangezogen. Dies kann zum Beispiel über maschinelles Lernen erfolgen. Aber auch klassische Verfahren, beispielsweise zur Berechnung der Inhomogenität, können dafür verwendet werden. Je höher der Fettgehalt (ermittelt aus der Dichte) und je weniger "glatt" die Textur (als Maß für den fibrotischen Umbau) ist, desto geringer ist die Abbaurate pro Volumeneinheit. Diese wird dann mit dem durch Segmentierung ermittelten Volumen der Leber multipliziert.

Die Metabolisierung und Elimination erfolgt über die Leber und einen kleineren Teil über die Lunge mit der Atemluft. Das Abatmen über die Lunge hängt vor allem von der Diffusionskapazität ab, die aus der Textur der Lunge abgeleitet werden kann. Bei größeren Lungenbläschen (Emphysem) ist die Diffusion geringer, also wird weniger Alkohol eliminiert. Außerdem spielt das Volumen und die Dichte der Lunge eine Rolle. Damit sind die Metabolisierung und Elimination relativ einfach berechenbar. Bei der Simulation kann insbesondere ermittelt werden, zu welcher Zeit welcher Blutalkoholspiegel erreicht ist. Umgekehrt kann mit Hilfe der Simulation ermittelt werden, zu welcher Zeit wieviel Alkohol aufgenommen wurde, wenn der Blutalkoholspiegel bekannt ist.

Diazepam wird vor allem hepatisch eliminiert, es ist sehr lipophil und hat daher ein sehr großes Verteilungsvolumen. Um die Distribution zu bestimmen, ist zunächst der Anteil des Fetts an der Körpermasse herauszufinden. Magnetresonanztomographie-Bilddaten sind dafür besonders vorteilhaft, speziell, wenn medizinische Bilder vorliegen, die mit einer Technik gemacht sind, die Fett und Wasser trennt, zum Beispiel eine inphase-opposed-phase-Technik.

Falls medizinische Bilder nur für einen bestimmten Körperbereich vorliegen (Körperstamm), kann vom Fettgehalt in diesen Bildern auf den Rest des Körpers hochgerechnet werden. Daraus lässt sich gut die Peak-Konzentration des Medikaments für jedes Organ berechnen, indem die Verteilung simuliert wird. Dies funktioniert sogar für das Gehirn, wenn es überhaupt nicht abgebildet ist, zum Beispiel basierend auf einem Abdomen-MRT. Dabei kann berücksichtigt werden, dass die Masse des Gehirns nur innerhalb eines schmalen Bandes von Patient zu Patient variiert.

Die Metabolisierung und Elimination von Diazepam kann analog zum Alkohol wie weiter oben beschrieben berechnet werden.

Falls auch Perfusionsdaten verfügbar sind, insbesondere für eine Hirnperfusion, kann auch die Umverteilung über die Zeit gut simuliert werden.

Im Folgenden werden Beispiele für insbesondere morphologische Eigenschaften genannt, für die jeweils ein Bildverarbeitungsergebnis ermittelt werden kann, welches für die Ermittlung des patientenspezifischen pharmakokinetischen Parameters verwendet werden kann.

Für die Absorption sind insbesondere eine Morphologie des Gastrointestinaltrakts, eine Länge des Darms und eine Dicke der Darmwand relevant. Ferner sind bei inhalierten Substanzen (Anästhetika) Form und Zustand der Atemwege sowie eine Textur der Lunge relevant. Für die Distribution sind insbesondere ein Fettgehalt, eine Fettmasse und deren Verteilung, eine Dicke des Unterhautfettgewebes, Perfusionen relevanter Organe, Kreislaufparameter (Herzfunktion etc.) und ein Gefäßzustand (Wanddicke, Anzeichen für Atherosklerose) relevant. Für die Metabolisierung sind insbesondere eine Lebertextur, ein Lebervolumen und eine Leberperfusion relevant. Für die Elimination sind insbesondere eine Morphologie der Harnwege (Steine, Blockaden, Stau), eine Morphologie und ggf. ein Fehlen der Nieren, eine Nierenperfusion (vor allem bei glomerulärer Elimination), eine Textur der Nieren und eine Ausscheidung von Kontrastmittel, insbesondere wenn die Ausscheidung auf den medizinischen Bildern erkennbar ist, zum Beispiel in Harnwegen und Blase.

Ferner können über die medizinischen Bilddaten hinaus weitere Patientendaten bereitgestellt und beim Ermitteln des patientenspezifischen pharmakokinetischen Parameters verwendet werden. Insbesondere können die Trainingsdatensätze zum Trainieren des Bilddatenverarbeitungsalgorithmus zusätzlich zu den medizinischen Bilddaten weitere Patientendaten aufweisen. Ein auf diese Weise trainierter Bilddatenverarbeitungsalgorithmus kann verwendet werden, um den patientenspezifischen pharmakokinetischen Parameter basierend auf den medizinischen Bilddaten und den weiteren Patientendaten zu ermitteln. Beispiele für weitere Patientendaten, sind in-vitro-Diagnosedaten, genetische Patientendaten, zum Beispiel P450-Varianten, Blutwerte, zum Beispiel Glutamat-Oxalacetat-Transaminase, physiologische Messwerte, zum Beispiel Körpergewicht, Spirometrie-Ergebnisse, Elektrokardiogramm etc. Die medizinischen Bilddaten und/oder die weiteren Patientendaten können insbesondere aus einer elektronischen Patientenakte geladen werden.

Die medizinischen Bilddaten können insbesondere aus einer medizinischen Bilddatenbank geladen werden und/oder mittels einer medizinischen Bildgebungsvorrichtung generiert werden. Die medizinische Bildgebungsvorrichtung kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MR-Gerät), einem Ultraschallbildgebungsgerät und Kombinationen daraus, insbesondere einem PET-CT-Gerät und einem PET-MR-Gerät, besteht.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Es zeigen:
Fig. 1 ein Ablaufdiagramm eines Verfahrens zum Ermitteln eines patientenspezifischen pharmakokinetischen Parameters,
Fig. 2 eine Datenverarbeitungseinheit zum Ermitteln eines patientenspezifischen pharmakokinetischen Parameters,
Fig. 3 ein Ablaufdiagramm eines Verfahrens zum Ermitteln eines patientenspezifischen Medikationsparameters,
Fig. 4 eine Datenverarbeitungseinheit zum Ermitteln eines patientenspezifischen Medikationsparameters,
Fig. 5 eine schematische Darstellung einer pharmakokinetischen Simulation basierend auf medizinischen Bilddaten, und
Fig. 6 eine medizinische Bildgebungsvorrichtung.

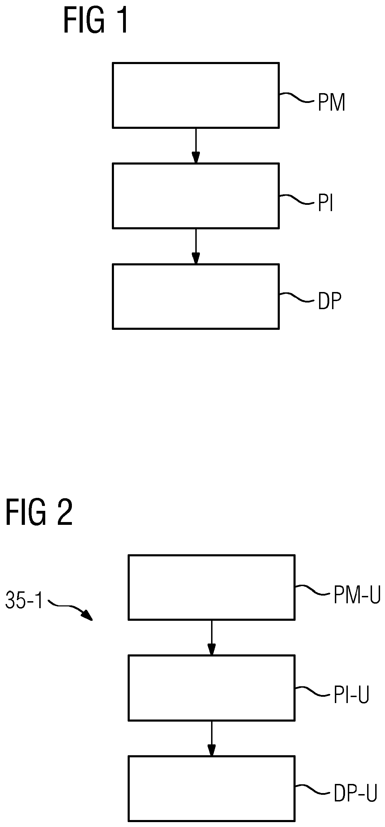

Die Fig. 1 zeigt ein Ablaufdiagramm eines Verfahrens zum Ermitteln eines patientenspezifischen pharmakokinetischen Parameters für ein Medikament, wobei das Verfahren die folgenden Schritte umfasst:
- Bereitstellen PM von allgemeinen pharmakokinetischen Daten des Medikaments,
- Bereitstellen PI von medizinischen Bilddaten MI eines Patienten 13,
- Ermitteln DP des patientenspezifischen pharmakokinetischen Parameters für das Medikament basierend auf den medizinischen Bilddaten MI und den allgemeinen pharmakokinetischen Daten des Medikaments.

Die Fig. 2 zeigt eine Datenverarbeitungseinheit 35-1 zum Ermitteln eines patientenspezifischen pharmakokinetischen Parameters, aufweisend:
- eine Pharmakokinetik-Daten-Bereitstellungseinheit PM-U, ausgebildet zum Bereitstellen PM von allgemeinen pharmakokinetischen Daten des Medikaments,
- eine Bilddaten-Bereitstellungseinheit PI-U, ausgebildet zum Bereitstellen PI von medizinischen Bilddaten MI eines Patienten 13,
- eine Parameter-Ermittlungseinheit DP-U, ausgebildet zum Ermitteln DP des patientenspezifischen pharmakokinetischen Parameters für das Medikament basierend auf den medizinischen Bilddaten MI und den allgemeinen pharmakokinetischen Daten des Medikaments.

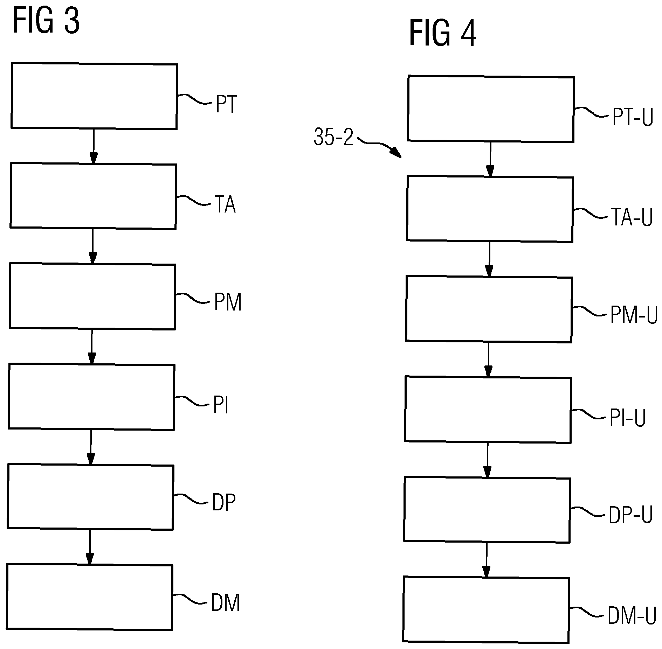

Die Fig. 3 zeigt ein Ablaufdiagramm eines Verfahrens zum Ermitteln eines patientenspezifischen Medikationsparameters für ein Medikament, wobei das Verfahren die folgenden Schritte umfasst:
- Bereitstellen PT eines Satzes von Trainingsdatensätzen, wobei jeder Trainingsdatensatz jeweils einen medizinischen Bilddatensatz und einen dem medizinischen Bilddatensatz zugeordneten pharmakokinetischen Parameter aufweist,
- Trainieren TA des Bilddatenverarbeitungsalgorithmus basierend auf dem Satz von Trainingsdatensätzen und einem Maschinenlernalgorithmus,
- Ermitteln zumindest eines patientenspezifischen pharmakokinetischen Parameters für das Medikament mittels des in der Fig. 1 gezeigten Verfahrens mit den Schritten PM, PI und DP, wobei der patientenspezifische pharmakokinetische Parameter für das Medikament unter Verwendung des trainierten Bilddatenverarbeitungsalgorithmus ermittelt wird,
- Ermitteln DM des patientenspezifischen Medikationsparameters basierend auf dem zumindest einen patientenspezifischen pharmakokinetischen Parameter und einem pharmakokinetischen Modell.

Die Fig. 4 zeigt eine Datenverarbeitungseinheit 35-2 zum Ermitteln eines patientenspezifischen Medikationsparameters, aufweisend:
- eine Trainingsdatensatz-Bereitstellungseinheit PT-U, ausgebildet zum Bereitstellen PT eines Satzes von Trainingsdatensätzen, wobei jeder Trainingsdatensatz jeweils einen medizinischen Bilddatensatz und einen dem medizinischen Bilddatensatz zugeordneten pharmakokinetischen Parameter aufweist,
- eine Bilddatenverarbeitungsalgorithmus-Trainingseinheit TAU, ausgebildet zum Trainieren TA des Bilddatenverarbeitungsalgorithmus basierend auf dem Satz von Trainingsdatensätzen und einem Maschinenlernalgorithmus,
- die Komponenten PM-U, PI-U und DP-U der in der Fig. 2 gezeigten Datenverarbeitungseinheit 35-1, wobei die Parameter-Ermittlungseinheit DP-U zum Ermitteln des patientenspezifischen pharmakokinetischen Parameters für das Medikament unter Verwendung des trainierten Bilddatenverarbeitungsalgorithmus ausgebildet ist,
- eine Medikationsparameter-Ermittlungseinheit DM-U, ausgebildet zum Ermitteln DM des patientenspezifischen Medikationsparameters basierend auf dem zumindest einen patientenspezifischen pharmakokinetischen Parameter und einem pharmakokinetischen Modell.

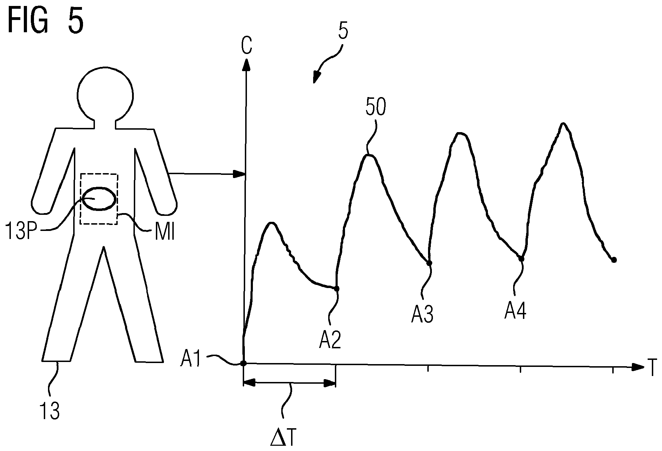

Die Fig. 5 zeigt eine schematische Darstellung einer pharmakokinetischen Simulation 5 basierend auf medizinischen Bilddaten MI. Bei den medizinischen Bilddaten MI kann es sich insbesondere um ein medizinisches Bild MI eines Organs 13P des Patienten 13 handeln. Basierend auf dem medizinischen Bild MI können ein oder mehrere pharmakokinetische Parameter ermittelt werden, die spezifisch für den Patienten 13 sind. Basierend auf diesen patientenspezifischen pharmakokinetischen Parametern und einem pharmakokinetischen Modell kann ein patientenspezifischer Verlauf 50 einer Konzentration C des Medikaments, beispielsweise im Blut des Patienten 13, über die Zeit T simuliert werden. An den Punkten A1, A2, A3, A4 wird das Medikament appliziert, insbesondere von dem Patienten 13 aufgenommen.

Basierend auf der Simulation 5 können ein oder mehrere patientenspezifische Medikationsparameter ermittelt werden, beispielsweise ein Zeitabstand ΔT zwischen zwei zeitlich benachbarten Medikamente-Applikationen A1, A2, A3, A4 und/oder eine Menge des Medikaments bei einer Medikamente-Applikation A1, A2, A3, A4. Die Zeitabstände zwischen zwei zeitlich benachbarten Medikamente-Applikationen A1, A2, A3, A4 und/oder die Menge des Medikaments bei einer Medikamente-Applikation A1, A2, A3, A4 können gleichbleibend sein oder speziell für jede Medikamente-Applikation A1, A2, A3, A4 optimiert werden.

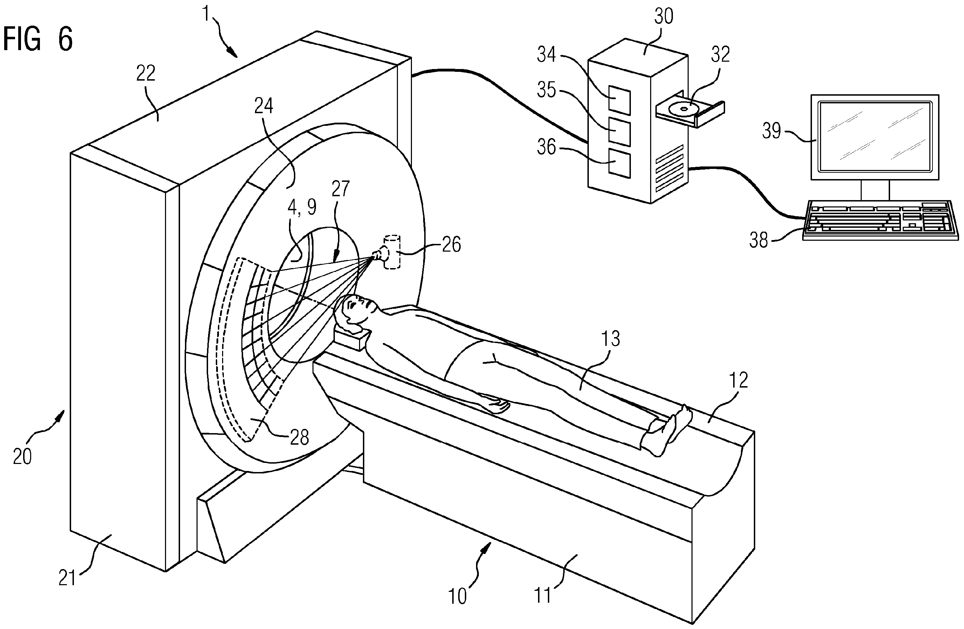

Die Fig. 6 zeigt eine medizinische Bildgebungsvorrichtung 1. Ohne Beschränkung des allgemeinen Erfindungsgedankens ist für die medizinische Bildgebungsvorrichtung 1 beispielhaft ein Computertomographiegerät gezeigt. Die medizinische Bildgebungsvorrichtung 1 weist die Gantry 20, die tunnelförmige Öffnung 9, die Patientenlagerungsvorrichtung 10 und die Steuerungsvorrichtung 30 auf. Die Gantry 20 weist den stationären Tragrahmen 21, den Kipprahmen 22 und den Rotor 24 auf. Der Kipprahmen 22 ist mittels einer Kipplagerungsvorrichtung an dem stationären Tragrahmen 21 relativ zu dem stationären Tragrahmen 21 kippbar angeordnet. Der Rotor 24 ist mittels einer Drehlagerungsvorrichtung an dem Kipprahmen 22 um eine Rotationsachse relativ zu dem Kipprahmen 22 drehbar angeordnet.

In die tunnelförmige Öffnung 9 ist der Patient 13 einführbar. In der tunnelförmigen Öffnung 9 befindet sich der Akquisitionsbereich 4. In dem Akquisitionsbereich 4 ist ein abzubildender Bereich des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann. Die Patientenlagerungsvorrichtung 10 weist den Lagerungssockel 11 und die Lagerungsplatte 12 zur Lagerung des Patienten 13 auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12 in den Akquisitionsbereich 4 einführbar ist.

Die medizinische Bildgebungsvorrichtung 1 ist zur Akquisition von Akquisitionsdaten basierend auf einer elektromagnetischen Strahlung 27 ausgebildet. Die medizinische Bildgebungsvorrichtung 1 weist eine Akquisitionseinheit auf. Die Akquisitionseinheit ist eine Projektionsdaten-Akquisitionseinheit mit der Strahlungsquelle 26, z. B. einer Röntgenquelle, und dem Detektor 28, z. B. einem Röntgendetektor, insbesondere einem energieauflösenden Röntgendetektor. Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Die Steuerungsvorrichtung 30 ist zum Empfangen der von der Akquisitionseinheit akquirierten Akquisitionsdaten ausgebildet. Die Steuerungsvorrichtung 30 ist zum Steuern der medizinischen Bildgebungsvorrichtung 1 ausgebildet. Die Steuerungsvorrichtung 30 weist die Datenverarbeitungseinheit 35, das computerlesbare Medium 32 und das Prozessorsystem 36 auf. Die Steuerungsvorrichtung 30, insbesondere die Datenverarbeitungseinheit 35, wird von einem Datenverarbeitungssystem, welches einen Computer aufweist, gebildet. Die allgemeinen pharmakokinetischen Daten des Medikaments können beispielsweise auf dem computerlesbaren Medium 32 gespeichert sein und/oder mittels der Steuerungsvorrichtung 30 von einem entfernten Speicherort heruntergeladen werden. Die Datenverarbeitungseinheit 35 weist die Datenverarbeitungseinheit 35-1 und/oder die Datenverarbeitungseinheit 35-2 auf.

Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz rekonstruiert werden. Die medizinische Bildgebungsvorrichtung 1 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, insbesondere medizinischen Bildern und/oder akustischen Signalen ausgebildet. Insbesondere kann mittels der Ausgabevorrichtung 39, beispielsweise in Form eines Bildschirms, die pharmakokinetische Simulation 5 dargestellt werden.

## Patentansprüche

1. Verfahren zum Ermitteln eines patientenspezifischen pharmakokinetischen Parameters für ein Medikament, wobei das Verfahren die folgenden Schritte umfasst:
- Bereitstellen (PM) von allgemeinen pharmakokinetischen Daten des Medikaments,
- Bereitstellen (PI) von radiologischen medizinischen Bilddaten (MI) eines Patienten (13),
- Ermitteln (DP) des patientenspezifischen pharmakokinetischen Parameters für das Medikament basierend auf den medizinischen Bilddaten (MI) und den allgemeinen pharmakokinetischen Daten des Medikaments,
- wobei ein Bildverarbeitungsergebnis basierend auf den medizinischen Bilddaten (MI) des Patienten (13) mittels eines Bilddatenverarbeitungsverfahrens ermittelt wird, wobei das Bildverarbeitungsergebnis eine Textur einer Leber betrifft,
- wobei der patientenspezifische pharmakokinetische Parameter basierend auf dem Bildverarbeitungsergebnis ermittelt wird, wobei der patientenspezifische pharmakokinetische Parameter für das Medikament eine Eliminationsgeschwindigkeit einer hepatischen Elimination des Medikaments in einer Reaktion nullter Ordnung ist,
- wobei der patientenspezifische pharmakokinetische Parameter für das Medikament unter Verwendung eines trainierten Bilddatenverarbeitungsalgorithmus ermittelt wird, wobei aus der Textur der Leber über maschinelles Lernen eine Elimination pro Volumeneinheit berechnet wird, wobei ein Verfahren des maschinellen Lernens verwendet wird, welches anhand von Bilddatensätzen mit bekannter Pharmakokinetik trainiert wurde,
- wobei mittels einer Segmentierung ein Gesamtvolumen der Leber bestimmt wird und
- wobei aus der Elimination pro Volumeneinheit und dem Gesamtvolumen der Leber der patientenspezifische pharmakokinetische Parameter für das Medikament ermittelt wird.

2. Verfahren nach Anspruch 1,
- wobei das Bilddatenverarbeitungsverfahren einen Bildverarbeitungsschritt umfasst, welcher aus der Gruppe gewählt ist, die aus der Segmentierung, einer Texturanalyse, einer Perfusionsbestimmung, einer Stoffwechselaktivitätsbestimmung und Kombinationen davon besteht.

3. Verfahren nach einem der Ansprüche 1 bis 2,
- wobei basierend auf den pharmakokinetischen Daten ein Körperbereich ausgewählt wird, welcher an einem pharmakokinetischen Prozess des Medikaments beteiligt ist,
- wobei das Bilddatenverarbeitungsverfahren basierend auf dem ausgewählten Körperbereich ausgewählt und/oder angepasst wird.

4. Verfahren nach einem der Ansprüche 1 bis 3,
- wobei die allgemeinen pharmakokinetischen Daten eine oder mehrere pharmakokinetische Eigenschaften des Medikaments betreffen, welche aus der Gruppe gewählt sind, die aus einer Applikation, einer Bioverfügbarkeit, einer Lipophilität, einem Verteilungsraum, einem Eliminationsweg und Kombinationen davon besteht.

5. Verfahren zum Ermitteln eines patientenspezifischen Medikationsparameters für ein Medikament, wobei das Verfahren die folgenden Schritte umfasst:
- Ermitteln zumindest eines patientenspezifischen pharmakokinetischen Parameters für das Medikament mittels eines Verfahrens nach einem der Ansprüche 1 bis 4,
- Ermitteln (DM) des patientenspezifischen Medikationsparameters basierend auf dem zumindest einen patientenspezifischen pharmakokinetischen Parameter und einem pharmakokinetischen Modell, insbesondere einem ADME-Modell.

6. Verfahren nach Anspruch 5,
- wobei der patientenspezifische Medikationsparameter ermittelt wird, indem ein patientenspezifischer zeitlicher Verlauf einer Konzentration des Medikaments simuliert wird.

7. Verfahren nach einem der Ansprüche 1 bis 6, wobei das Verfahren die folgenden Schritte umfasst:
- Bereitstellen (PT) eines Satzes von Trainingsdatensätzen, wobei jeder Trainingsdatensatz jeweils einen medizinischen Bilddatensatz und einen dem medizinischen Bilddatensatz zugeordneten pharmakokinetischen Parameter aufweist,
- Trainieren (TA) des Bilddatenverarbeitungsalgorithmus basierend auf dem Satz von Trainingsdatensätzen und einem Maschinenlernalgorithmus.

8. Datenverarbeitungseinheit, ausgebildet zum Ausführen eines Verfahrens nach einem der Ansprüche 1 bis 7.

9. Computerprogrammprodukt mit einem Computerprogramm, welches direkt in eine Speichereinrichtung eines Computers (30) ladbar ist, mit Programmabschnitten, um alle Schritte eines Verfahrens nach einem der Ansprüche 1 bis 7 auszuführen, wenn das Computerprogramm in dem Computer (30) ausgeführt wird.

10. Computerlesbares Medium (32), auf welchem von einem Computer (30) einlesbare und ausführbare Programmabschnitte gespeichert sind, um alle Schritte eines Verfahrens nach einem der Ansprüche 1 bis 7 auszuführen, wenn die Programmabschnitte von dem Computer (30) ausgeführt werden.

## Claims

1. Method for determining a patient-specific pharmacokinetic parameter for a drug, wherein the method comprises the following steps:
- providing (PM) general pharmacokinetic data of the drug,
- providing (PI) radiological medical image data (MI) of a patient (13),
- determining (DP) the patient-specific pharmacokinetic parameter for the drug based on the medical image data (MI) and the general pharmacokinetic data of the drug,
- wherein an image processing result is determined based on the medical image data (MI) of the patient (13) by means of an image data processing method, wherein the image processing result relates to a texture of a liver,
- wherein the patient-specific pharmacokinetic parameter is determined based on the image processing result, wherein the patient-specific pharmacokinetic parameter for the drug is an elimination speed of a hepatic elimination of the drug in a zeroth-order reaction,
- wherein the patient-specific pharmacokinetic parameter for the drug is determined using a trained image data processing algorithm, wherein an elimination per volume unit is calculated from the texture of the liver by way of machine learning, wherein a method of machine learning is used, which has been trained on the basis of image data records with known pharmacokinetics,
- wherein an overall volume of the liver is ascertained by means of a segmentation and
- wherein the patient-specific pharmacokinetic parameter for the drug is determined from the elimination per volume unit and the overall volume of the liver.

2. Method according to claim 1,
- wherein the image data processing method comprises an image processing step, which is selected from the group consisting of the segmentation, a texture analysis, a perfusion determination, a metabolic activity determination, and combinations thereof.

3. Method according to one of claims 1 to 2,
- wherein a body region which forms part of a pharmacokinetic process of the drug is selected based on the pharmacokinetic data,
- wherein the image data processing method is selected and/or adjusted based on the selected body region.

4. Method according to one of claims 1 to 3,
- wherein the general pharmacokinetic data relates to one or more pharmacokinetic properties of the drug, which are selected from the group consisting of an application, a bioavailability, lipophilicity, a distribution area, an elimination path, and combinations thereof.

5. Method for determining a patient-specific medication parameter for a drug, wherein the method comprises the following steps:
- determining at least one patient-specific pharmacokinetic parameter for the drug by means of a method according to one of claims 1 to 4,
- determining (DM) the patient-specific medication parameter based on the at least one patient-specific pharmacokinetic parameter and a pharmacokinetic model, in particular an ADME model.

6. Method according to claim 5,
- wherein the patient-specific medication parameter is determined by a patient-specific temporal course of a concentration of the drug being simulated.

7. Method according to one of claims 1 to 6, wherein the method comprises the following steps:
- providing (PT) a set of training data records, wherein each training data record has in each case a medical image data record and a pharmacokinetic parameter assigned to the medical image data record,
- training (TA) the image data processing algorithm based on the set of training data records and a machine learning algorithm.

8. Data processing unit, embodied to carry out a method according to one of claims 1 to 7.

9. Computer program product with a computer program, which can be loaded directly into a storage facility of a computer (30), with program segments, in order to carry out all steps of a method according to one of claims 1 to 7, when the computer program is executed in the computer (30).

10. Machine-readable medium (32), on which program segments which can be read in and executed by a computer (30) are stored, in order to execute all steps of a method according to one of claims 1 to 7, when the program segments are executed by the computer (30).

## Revendications

1. Procédé de détermination d'un paramètre pharmacocinétique, spécifique à un patient, d'un médicament, le procédé comprenant les stades suivants :
- on se procure (PM) des données pharmacocinétiques générales du médicament,
- on se procure (PI) des données (MI) d'images médicales radiologiques d'un patient (13),
- on détermine (DP) le paramètre pharmacocinétique, spécifique au patient, du médicament sur la base des données (MI) d'images médicales et des données pharmacocinétiques générales du médicament,
- dans lequel on détermine au moyen d'un procédé de traitement de données d'images un résultat de traitement d'images sur la base des données (MI) d'images médicales du patient (13), le résultat de traitement d'images concernant la texture d'un foie,
- dans lequel on détermine le paramètre pharmacocinétique spécifique au patient sur la base du résultat de traitement d'images, le paramètre pharmacocinétique, spécifique au patient, du médicament étant une vitesse d'une élimination hépatique du médicament dans une réaction d'ordre zéro,
- dans lequel on détermine le paramètre pharmacocinétique, spécifique au patient, du médicament en utilisant un algorithme de traitement de données d'images ayant subi un apprentissage, dans lequel on calcule à partir de la texture du foie par un apprentissage automatique, une élimination par unité de volume, dans lequel on utilise un procédé d'apprentissage automatique, qui a subi un apprentissage à l'aide d'ensembles de données d'images de pharmacocinétique connue,
- dans lequel on détermine un volume d'ensemble du foie au moyen d'une segmentation et
- dans lequel on détermine à partir de l'élimination par unité de volume et du volume total du foie le paramètre pharmacocinétique, spécifique au patient, du médicament.

2. Procédé suivant la revendication 1,
- dans lequel le procédé de traitement de données d'images comprend un stade de traitement d'images choisi dans le groupe constitué de la segmentation, d'une analyse de texture, d'une détermination de perfusion, d'une détermination d'activité de métabolisme et de leurs combinaisons.

3. Procédé suivant l'une des revendications 1 à 2,
- dans lequel on choisit sur la base des données pharmacocinétiques une partie du corps qui prend part à un processus pharmacocinétique du médicament,
- dans lequel on choisit et/ou on adapte le procédé de traitement de données d'images sur la base de la partie du corps choisie.

4. Procédé suivant l'une des revendications 1 à 3,
- dans lequel les données pharmacocinétiques générales concernent une ou plusieurs propriétés pharmacocinétiques du médicament choisies dans le groupe constitué d'une application, d'une biodisponibilité, d'une lipophilité, d'un espace de répartition, d'une voie d'élimination et de leurs combinaisons.

5. Procédé de détermination d'un paramètre de médication, spécifique à un patient, d'un médicament, le procédé comprenant les stades suivants :
- détermination d'au moins un paramètre pharmacocinétique, spécifique au patient, du médicament au moyen d'un procédé suivant l'une des revendications 1 à 4,
- détermination (DM) du paramètre de médication, spécifique au patient, sur la base du au moins un paramètre pharmacocinétique, spécifique au patient, et d'un modèle pharmacocinétique, notamment d'un modèle ADME.

6. Procédé suivant la revendication 5,
- dans lequel on détermine la paramètre de médication, spécifique au patient, en simulant une courbe en fonction du temps, spécifique au patient, d'une concentration du médicament.

7. Procédé suivant l'une des revendications 1 à 6, dans lequel le procédé comprend les stades suivants :
- mise à disposition d'un ensemble d'ensembles de données d'apprentissage, chaque ensemble de données d'apprentissage ayant respectivement un ensemble de données d'images médicales et un paramètre pharmacocinétique associé à l'ensemble de données d'images médicales,
- apprentissage (TA) de l'algorithme de traitement de données d'images reposant sur l'ensemble des ensembles de données d'apprentissage et sur un algorithme d'apprentissage automatique.

8. Unité de traitement de données constituée pour effectuer un procédé suivant l'une des revendications 1 à 7.

9. Produit de programme d'ordinateur ayant un programme d'ordinateur, qui peut être chargé directement dans un dispositif de mémoire d'un ordinateur (30), comprenant des parties de programme pour effectuer tous les stades d'un procédé suivant l'une des revendications 1 à 7, lorsque le programme d'ordinateur est exécuté dans l'ordinateur (30).

10. Support (12) déchiffrable par ordinateur, sur lequel des parties de programme déchiffres par l'ordinateur et pouvant être exécutées sont mises en mémoire pour effectuer tous les stades d'un procédé suivant l'une des revendications 1 à 7, lorsque les parties du programme sont exécutées par l'ordinateur (30).
